Europäisches Patentamt

European Patent Office

Office européen des brevets

(11) Publication number: **0 180 597 B1**

# EUROPEAN PATENT SPECIFICATION

(45) Date of publication of patent specification: **04.03.92**

(51) Int. Cl.5: **A61K 45/06**, A61K 31/19, A61K 31/44, A61K 31/485, //(A61K31/19,31:135), (A61K31/44,31:19), (A61K31/485,31:19)

(21) Application number: **85901895.4**

(22) Date of filing: **08.04.85**

(86) International application number:
**PCT/US85/00596**

(87) International publication number:
**WO 85/04589 (24.10.85 85/23)**

The file contains technical information submitted after the application was filed and not included in this specification

(54) **COUGH/COLD MIXTURES COMPRISING NON-STEROIDAL ANTI-INFLAMMATORY DRUGS.**

(30) Priority: **09.04.84 US 598502**

(43) Date of publication of application:
**14.05.86 Bulletin 86/20**

(45) Publication of the grant of the patent:
**04.03.92 Bulletin 92/10**

(84) Designated Contracting States:
**AT BE CH DE FR GB IT LI LU NL SE**

(56) References cited:
**EP-A- 0 097 953**
**US-A- 4 322 427**

**European Journal of Pharmacology 82 (1982) 121 - 130; Vergafting et al**

**UNLISTED DRUGS, vol. 20, nr. 11, November 1968, (Chatham, New Jersey, US) p. 169n "SINUBID"**

(73) Proprietor: **RICHARDSON-VICKS, INC.**
**One Far Mill Crossing**
**Shelton, Connecticut 06484(US)**

(72) Inventor: **Sunshine, Abraham**
**45 East 80th Street**
**New York, NY 10021(US)**
Inventor: **Laska, Eugene M.**
**34 Dante Street**
**New York, NY 10538(US)**
Inventor: **Siegel, Carole E.**
**1304 Colonial Court**
**Mamaroneck, NY 10532(US)**

(74) Representative: **Pendlebury, Anthony et al**
**PAGE, WHITE & FARRER 54 Doughty Street**
**London WC1N 2LS(GB)**

Rank Xerox (UK) Business Services

UNLISTED DRUGS, vol. 23, nr. 3, March 1971, (Chatham, New Jersey, US) p. 36, "CO-TYLENOL"

Chemical Abstracts, vol. 101, nr. 23, 3 December 1984, (Columbus, Ohio, US) TRABER, Daniel L.: "Ibuprofen and diphenhydramine reduce the lung lesion of endotoxemia in sheep" p. 39, column 1, abstract nr. 204101x

## Description

### Background of the Invention

The present invention relates generally to novel pharmaceutical compositions of matter comprising one or more non-steroidal anti-inflammatory drugs (NSAID) in combination with at least one antihistamine, sympathomimetic drug (nasal decongestant, bronchodilator), non-narcotic cough suppressant and/or expectorant, optionally in combination with suitable pharmaceutically acceptable non-toxic carriers or excipients, and to methods of using said compositions in the treatment, management or mitigation of cough, cold, cold-like and/or flu symptoms and the discomfort, pain, fever and general malaise associated therewith.

Non-narcotic analgesics, most of which are also known as non-steroidal anti-inflammatory drugs (NSAID), are widely administered orally in the treatment of mild to severe pain. Within this class, the compounds vary widely in their chemical structure and in their biological profiles as analgesics, anti-inflammatory agents and antipyretic agents. Among the most commonly used members of the non-narcotic analgesic class of drugs are aspirin, acetaminophen and phenacetin. Aspirin and acetaminophen have heretofore been included as the pain reliever and fever-reducing component in conventional cough/cold multi- symptom alleviating compositions.

However, a number of alternative non-narcotic agents offering a variety of advantages over these conventionally employed non-narcotic analgesic antipyretics have now been developed. The principal advantages of these non-steroidal anti-inflammatory drugs include not only the clinically superior analgesic, anti-inflammatory and antipyretic activity of these agents compared to aspirin, acetaminophen or phenacetin, but also a minimization of the adverse side effects experienced with these conventional agents; more specifically, the gastrointestinal ulcerations experienced with aspirin and the hepatic toxicity prevalent with the chronic use of acetaminophen.

Exemplary prior art cough/cold formulations containing aspirin® or acetaminophen include Coricidin®, Coricidin D®, Comtrex®, Dristan®, Daycare®, Cotylenol®, Sinubid® and the like. These formulations generally contain in addition to aspirin or acetaminophen, one or more antihistaminics, decongestants, cough suppressants, antitussives and expectorants.

While aspirin and acetaminophen have been utilized in these previous compositions, it has not been heretofore proposed to use any of the newer non-steroidal anti-inflammatory drugs (i.e., excluding aspirin, acetaminophen and phenacetin) in the preparation of advantageous cough/cold pharmaceutical compositions.

### Summary of the Invention

It is, therefore, a primary object of the present invention to provide pharmaceutical compositions of matter for use in the treatment of cough, cold-like and/or flu symptoms in a mammalian organism, and adapted for unit dosage oral administration, said composition comprising (i) at least one non-narcotic analgesic constituent which is a non-steroidal anti-inflammatory drug (NSAID) selected from a propionic acid derivative, an acetic acid derivative, a fenamic acid derivative, a biphenylcarboxylic acid derivative, an oxicam, or a pharmaceutically acceptable salt thereof (excluding acetylsalicylic acid (aspirin®), acetaminophen and phenacetin), in combinatory admixture with (ii) at least one of (a) at least one antihistamine other than $\alpha$-fluoromethylhistidine, mepyramine or diphenhydramine, (b) at least one sympathomimetic decongestant or bronchodilator, (c) at least one expectorant, and (d) at least one non-narcotic antitussive (cough suppressant), or pharmaceutically acceptable salts thereof.

Further objects of the present invention are defined in the claims.

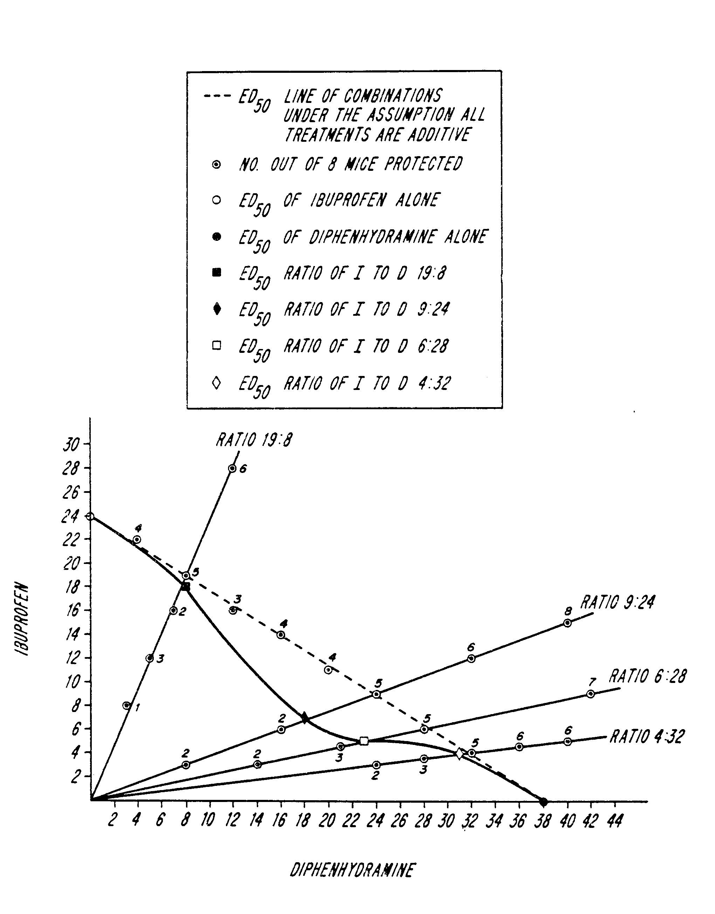

### Brief Description of the Drawing

The Figure of Drawing is a plot of dose of diphenhydramine versus dose of ibuprofen in the phenylquinone writhing assay to indicate the number of mice protected.

### Detailed Description of the Invention

More specifically, the applicants herein have found that certain non-steroidal anti-inflammatory agents are ideally suited for use in cough/cold formulations by reason of their enhanced analgesic anti-inflammatory and antipyretic activity and low incidence of untoward side effects, particularly at the optimum dosages provided for in the present invention, compared to aspirin or acetaminophen.

The superiority of various of the non-narcotic analgesics belonging to the non-steroidal anti-inflammatory drug class in comparative studies with aspirin and acetaminophen is well documented in the literature.

Cooper in 1977 found that ibuprofen 400 mg had a greater peak effect and longer duration of action than aspirin 650 mg. Cooper, S.A., Needle, A.E., Kruger, G.O. 1977. "An Analgesic Relative Potency Assay Comparing Aspirin, Ibuprofen and Placebo. "J. Oral Surg. 35:898-903. Cooper in another study in 1982 found 400 mg of ibuprofen to be more effective than aspirin 650 mg. Cooper, S.A., Engel, J., Ladov, M., Precheur, H., Rosenheck, A., Rauch, D. 1982. "Analgesic Efficacy of an Ibuprofen-codeine Combination." Pharmacotherapy 2:162-67. Sunshine et al found ibuprofen to be significantly superior to aspirin in the relief of post-episiotomy pain. Sunshine, A. et al, Clinical Pharmacology and Therapeutics, :24:254-250, 1983.

Dionne in 1982 found ibuprofen to be more effective than acetaminophen in delaying the onset and intensity of post-operative dental pain. Dionne, R.A., Campbell, R.A., Cooper, S.A., Hall, D.L., Buckingham, B. "Suppression of Post operative Pain by Preoperative Administration of Ibuprofen in Comparison to Placebo, Acetaminophen and Acetaminophen Plus Codeine." J. Clin. Phamacol. (In press).

Naproxen sodium 550 mg was compared with 650 mg of aspirin and was found to provide earlier and better pain relief than aspirin by Sevelius, H., J. Clin. Pharmacol. 20:480-485, 1980. "Comparative Analgesic Effects of Naproxen Sodium, Aspirin and Placebo."

Flurbiprofen 50 and 100 mg was significantly more effective than aspirin 600 mg. Flurbiprofen 25 mg was slightly less effective than aspirin 600 mg. Sunshine, A., Olson N.Z., Laska, E.M. Zighelboim, I., DeCastro, A., Desarrazin, C., Pharmaco Ther. 3:177-181. " Analgesic Effect of Graded Doses of Flurbiprofen in Postepisiotomy Pain".

Silberman found suprofen 200 mg more effective than aspirin 650 mg for pain relief in the treatment of moderate to severe pain resulting from musculoskeletal pain. Silberman, H.M. "Multiple-Dose Comparison of Suprofen, Aspirin and Placebo in the Treatment of Musculoskeletal Pain." Pharmacology 27:S 1, 65-73 (1983).

While these reported findings with respect to the outstanding analgesic properties of the non-steroidal anti-inflammatory drugs compared to aspirin or acetaminophen have prompted the widespread acceptance and usage of these newer non-narcotic analgesics, as single entities, for the treatment and management of acute and chronic inflammatory states, notably rheumatoid arthritis and osteoarthritis, the utilization of these agents in cough/cold compositions has not heretofore been considered.

The non-steroidal anti-inflammatory drugs (NSAID's) for use in the pharmaceutical compositions and methods of use of the present invention may be selected from any of the following categories:

(1) The propionic acid derivatives;

(2) The acetic acid derivatives;

(3) The fenamic acid derivatives;

(4) The biphenylcarboxylic acid derivatives; and

(5) The oxicams.

Accordingly, the term "NSAID" as used herein is intended to mean any non-narcotic analgesic non-steroidal anti-inflammatory compound, including the pharmaceutically acceptable non-toxic salts thereof, falling within one of the five structural categories above but excluding aspirin, acetaminophen and phenacetin.

The specific compounds falling within the foregoing definition of the non-steroidal anti-inflammatory drugs for use in the present invention are well known to those skilled in the art and reference may be had to various literature reference sources for their chemical structures, pharmacological activities, side effects, normal dosage ranges, etc. See, for example, Physician's Desk Reference, 35th Edition, 1981 and The Merck Index, 9th Edition, Merck and Company, Rahway, New Jersey (1976) and Cutting's Handbook of Pharmacology, 6th Edition, Ed. T. Z. Csacky, M.D., Appleton-Century-Crofts, New York, 1979, Chapter 49:538-550.

Of the propionic acid derivatives for use herein, ibuprofen, naproxen, flurbiprofen, fenoprofen, ketoprofen, suprofen, fenbufen, and fluprofen may be mentioned as particularly preferred compounds.

Of the acetic acid derivatives, presently preferred members include tolmetin sodium, zomepirac, sulindac and indomethacin.

Of the fenamic acid derivatives, particularly preferred compounds include mefenamic acid and meclofenamate sodium.

The particularly preferred biphenylcarboxylic acid derivatives for use in the present invention include diflunisal and flufenisal.

The particularly advantageous oxicams include piroxicam, sudoxicam and isoxicam.

Of course, it will be appreciated by those skilled in the art, that any of the foregoing compounds may

4

be utilized in the form of their pharmaceutically acceptable salt forms, e.g., -COO⁻Na⁺, -COO⁻K⁺, and the like.

Of the foregoing non-steroidal anti-inflammatory drugs, in the practice of the preferred embodiments of the present invention, ibuprofen and naproxen are most preferred.

With respect to the dosage amount of the non-steroidal anti-inflammatory drugs in the compositions of the invention, although the specific dose will vary depending upon the age and weight of the patient, the severity of the symptoms, the incidence of side effects and the like, for humans, typical effective analgesic amounts of presently preferred NSAID's for use in unit dose compositions of the invention are about 100 - 500 mg diflunisal, about 25 - 100 mg zomepirac sodium, about 50-400 mg ibuprofen, most preferably 100-200 mg, about 125-500 mg naproxen, about 25-100 mg flurbiprofen, about 50-100 mg fenoprofen, about 10-20 mg piroxicam, about 125-250 mg mefenamic acid, about 100-400 mg fenbufen or about 25-50 mg ketoprofen; however, greater or lesser amounts may be employed if desired or necessary. With respect to the compounds set forth hereinabove falling within the propionic acid derivative category, suitable dosage ranges for these compounds will generally fall within the range of 25 mg to 600 mg in each unit dose.

A complete description of the various NSAID's, including acceptable analgesically effective amounts thereof for use in unit dose compositions of the present invention also appears in applicants United States Patent No. 4,486,436.

The cough/cold pharmaceutical compositions of the present invention comprise, in addition to the non-steroidal anti-inflammatory drugs, at least one active ingredient from the following pharmacological classes: antihistamines, sympathomimetics (decongestants), non-narcotic antitussives (cough suppressants), and expectorants. Typical therapeutically active components from these categories, along with their usual adult dosage, for use in the pharmaceutical compositions and methods of the invention are set forth in the following Table 1.

## TABLE I

| DRUG (FORM-SALT) | ACTION[1] | PREPARATIONS | USUAL SINGLE DOSE (ADULT) |
|---|---|---|---|
| chlorpheniramine (maleate) | A | Tablets, Capsules, 4 mg, 8 mg, 12 mg, (substained Action) 12 mg | 2-4 mg |
| brompheniramine (maleate) | A | Tablets, Capsules, 4 mg, 8 mg, 12 mg (Extentabs R ) | 8-12 mg |
| dexchlorpheniramine (maleate) | A | Tablets, 2 mg, 4 mg, 6 mg, Syrup, Expectorant (2mg/5cc) | 2-6 mg |
| dexbrompheniramine (maleate) | A | Tablet, 6 mg | 6 mg |
| triprolidine (HCl) | A | Tablet, 2.5 mg. Syrup - 1.25 mg/5cc | 1.25-2.5 mg |
| diphenhydramine (HCl) | A | Tablets, Capsules, Elixir, Parenteral, 25 mg, 50 mg 12.5 mg/5cc; 10-50 mg/ml. | 12.5-50 mg |
| doxylamine (succinate) | A | Tablets, Elixir 10 mg, 7.5 mg/10cc., | 7.5 - 10 mg |
| tripelennamine (HCl) | A | Tablet, Elixir, 25 mg, 50 mg, 37.5 mg/5cc. | 25 - 50 mg. |
| cyproheptadine (HCl) | A | Tablet, Syrup, 4 mg, 1mg/5cc | 4 mg. |
| carbinoxamine (maleate) | A | Syrup 4mg/5cc., | 4 mg. |
| bromodiphenhydramine (HCl) | A | Syrup 3.75 mg/5cc | 3.75 mg. |

6

## TABLE I (continued)

| DRUG (FORM-SALT) | ACTION | PREPARATIONS | USUAL SINGLE DOSE (ADULT) |
|---|---|---|---|
| phenindamine (tartrate) | A | Tablet, Elixir 10 mg, 5 mg/5cc. | 10 mg. |
| pyrilamine (maleate, tannante) | A | Tablet 12.5 mg. | 12.5 mg. |
| azatadine (maleate) | A | Tablet, 1 mg. | 1 - 2 mg. |
| pseudoephedrine (HCl) | D | Tablet, Capsule 30 mg, 60 mg, 120 mg (sustained action) | 60 - 120 mg. |
| phenylpropanolamine (HCl) | D | Tablet, Capsule, Elixir, 25 mg, 50 mg, 12.5 mg/5cc | 25 - 50 mg. |
| phenylephrine (bitartrate, tannate, HBr, HCl) | D | Tablet, Capsule Elixir, 5 mg, 10 mg, 25 mg, 5 mg/5cc. | 5-25 mg. |
| caramiphen (edisylate) | CS | Capsule, Elixir 20 mg, 5mg/5cc | 5-20 mg. |
| dextromethorphan (HBr) | CS | Tablet, Capsule Elixir 15 mg. 30 mg. 15 mg/5cc. | 30 mg. |
| codeine (phosphate, sulfate) | CS | Tablet, Elixir 10 mg, 10 mg/5cc. | 10 mg. |
| terpin hydrate | E | Tablet 300 mg. | 300 mg. |
| guaifenesin (glyceryl guaiacolate) | E | Tablet, Capsule Elixir, 100 mg, 100 mg/5cc. | 100 mg. |

TABLE I (continued)

| DRUG (FORM-SALT) | ACTION | PREPARATIONS | USUAL SINGLE DOSE (ADULT) |
|---|---|---|---|
| potassium (Iodide, citrate) | E | Tablet, Elixir, 100 mg, 100 mg/5cc. | 150-300 mg. |
| potassium guaicolsulfonate | E | Elixir 80 mg/5cc. | 80 mg |

[1] A = antihistamine
D = decongestant
CS= cough suppressant
E = expectorant

Among such Table 1 antihistamines, sympathomimetics, cough suppressants-antitussives and expectorants, in combination with a non-steroidal anti-inflammatory drug, applicants have already demonstrated a synergistically enhanced analgesic and anti-inflammatory response in a mammalian organism, as shown below in Example 1.

Example 1 - Pharmacologic Test for Synergism - Ibuprofen/Diphenhydramine.

The unexpected synergistic analgesic effect of the addition of diphenhydramine to ibuprofen is evidenced by tests conducted on mice. Blue Spruce Farm male mice weighing 18-28 grams at the time of testing are used throughout. All mice are dosed orally by gavage with ibuprofen and/or diphenhydramine. The formulation of each test article is a solution or suspension in 0.25% methylcellulose manufactured by Fisher Scientific Company. A dozing volume of 10 ml/mg is used. All doses are coded and the test is performed under a code not known to the observer. Doses are based upon the weights of the animal taken prior to dosing.

METHOD

A phenylquinone writhing assay in mice was conducted over a four day period to test for synergism of the analgesic activity of ibuprofen and diphenhydramine.

The assay consists of phenyl-p-benzoquinone (PPQ) introduced in mice thirty minutes post dose of the test treatment(s). The PPQ is prepared as a .02% aqueous solution in 5 ml ethyl alcohol q.s. to 100 ml with distilled water and is administered intraperitoneally at .25 ml/mouse. The mice are injected with the PPQ solution and are placed in individual plastic squares 4"x4"x5" deep and observed for a ten minute period post treatment dose for exhibition of the writhing syndrome. Complete blocking of the writhing syndrome for the ten minute observation period in any one mouse is considered a positive response for that mouse. Conversely, if the mouse definitely writhes at least once, it is considered to be not protected from the PPQ.

Three hundred twenty-eight mice were randomly assigned to 40 groups. Two groups of ten mice per series were assigned to a control group (10 prior to the administration of the test treatments and 10 post administration) to verify the ability of the solutions to produce the writhing response.

The purpose of the assay on the first day is to estimate the $ED_{50}$ (effective dose in 50% of treated mice) of ibuprofen alone and of diphenhydramine alone, and to estimate the relative potency, , of ibuprofen to diphenhydramine, determined as the ratio of the $ED_{50}$ of ibuprofen to the $ED_{50}$ of diphenhydramine. Eight mice per group are dosed orally (via intubation) with 2, 5, 10 and 20 mg/kg of ibuprofen and 5, 10, 20 and 50 mg/kg of diphenhydramine. Table 2 shows the number of mice protected from writhing activity for each dose of ibuprofen and diphenhydramine. The method of Finney ["Statistical Method of Biological Assay", McMillan Pub., 3rd Edition, 1978] is used to estimate the $ED_{50}$'s of ibuprofen alone and diphenhydramine alone.

On the second day eight combination doses were studied. The doses were chosen based upon the $ED_{50}$'s established in the preceding day's experiment, which, under the assumption of additivity, would provide protection for 50% of the mice. These doses were tested in order to observe those ratio(s) of the combination drugs that would yield a synergistic effect. Combinations for which five or more mice exhibit blockage of writing are candidates for further study. The doses of the constituent drugs in mg/kg for the eight groups were for ibuprofen (I) and diphenhydramine (D) respectively, [abbreviated as (I,D)]: (22,4), (19,8), (16,12), (14,6), (11,20), (9,24), (6,28), (4,32). Table 3 shows for each of these combination doses, the number of mice protected from writhing activity.

On the third and fourth days the four specific fixed ratios that achieved 5 or more protected mice were studied in more detail, i.e., the first combination treatment used a ratio of ibuprofen to diphenhydramine of 19:8 and the doses of the constituent drugs in mg/kg that were studied were (8,3), (12,5), (16,7) and (28,12). The second combination treatment used a ratio of doses of ibuprofen to diphenhydramine of 6:28 and the doses of the constituent drugs in mg/kg that were studied were (3,14), (4.5,21) and (9,42). The third combination treatment used a ratio of doses of ibuprofen to diphenhydramine of 9:24 and the doses of the constituent drugs in mg/kg that were studied were (3,8), (6,16), (12,32) and (15,40). The fourth combination treatment used a ratio of doses of ibuprofen to diphenhydramine of 4:32 and the doses of the constituent drugs in mg/kg that were studied were (3,24), (3.5,28) (4.5,36) and (5,40).

Under the assumption of additivity each dose of each combination is equivalent to a dose of ibuprofen, based on the relative potency ($\rho$) of diphenhydramine to ibuprofen obtained from the experiment on the first day. Thus, for example, in the dose ratio 19:8 the combination of 28 mg/kg of ibuprofen and 12 mg/kg of diphenhydramine is, under the assumption of additivity, equivalent to $(28 + 12\rho)$ mg/kg of ibuprofen. Table 4 shows for each dose of each of the combination doses tested the number of mice observed to be protected and the ibuprofen equivalent dose. For each of the four combination ratios, $ED_{50}$'s were estimated based on the observed number of mice protected at each ibuprofen equivalent dose using the method of Finney. Table 5 displays the estimated $ED_{50}$'s for each ratio.

## RESULTS

The surprising synergistic effects of combining ibuprofen with diphenhydramine can be seen from the results of Tables 4 and 5 and the Figure of Drawing. The Figure of Drawing summarizes all of the findings by depicting the $ED_{50}$'s obtained for each treatment alone, the $ED_{50}$ line if the treatments were additive, the number of mice/protected from writhing for each treatment studied and the estimated $ED_{50}$'s for each combination ratio.

The $ED_{50}$ of ibuprofen alone is estimated to be 24 mg/kg and for diphenhydramine to be 38 mg/kg. The relative potency of diphenhydramine to ibuprofen is 24/38. Among the 8 ratios tested on the second day, synergism appears to be present for four ratios, and these ratios were further investigated on days 3 and 4. The $ED_{50}$'s were found to be for the dosage ratio of 19:8, 23 mg/kg of ibuprofen, for the dosage ratio 6:28, 19 mg/kg of ibuprofen, for the dosage ratio 9:24, 18 mg/kg of ibuprofen, and for the dosage ratio 4:32, 23 mg/kg of ibuprofen. Two of these $ED_{50}$'s are substantially less than 24 mg/kg of ibuprofen which is the $ED_{50}$ that would be expected if the effects were additive. This represents a 25% reduction of the amount of ibuprofen that is required to obtain the effect in 50% of the animals. The Figure of Drawing indicates that many other dose ratios as well would produce an unexpected synergistic effect.

9

## TABLE 2

NUMBER OF MICE PROTECTED AT TESTED DOSE LEVELS

OF IBUPROFEN AND DIPHENHYDRAMINE

| Dose of Ibuprofen mg/kg | Dose of Diphenhydramine mg/kg | Number of Mice Protected | Number of Mice Not Protected |
|---|---|---|---|
| 2 | - | 0 | 8 |
| 5 | - | 0 | 8 |
| 10 | - | 1 | 7 |
| 20 | - | 3 | 5 |
| - | 5 | 1 | 7 |
| - | 10 | 2 | 6 |
| - | 20 | 3 | 5 |
| - | 40 | 4 | 4 |

EP 0 180 597 B1

## TABLE 3

NUMBER OF MICE PROTECTED AT TESTED DOSES* OF THE COMBINATION

OF IBUPROFEN AND DIPHENHYDRAMINE

| Dose of Ibuprofen mg/kg | Dose of Diphenhydramine mg/kg | Number of Mice Protected | Number of Mice Not Protected |
|---|---|---|---|
| 22 | 4 | 4 | 4 |
| 19 | 8 | 5 | 3 |
| 16 | 12 | 3 | 5 |
| 14 | 16 | 4 | 4 |
| 11 | 20 | 4 | 4 |
| 9 | 24 | 5 | 3 |
| 6 | 28 | 5 | 3 |
| 4 | 32 | 5 | 3 |

* Doses were chosen based upon $ED_{50}$'s of ibuprofen and diphenhydramine which under the assumption of additivity would provide protection for 50% of the mice.

EP 0 180 597 B1

## TABLE 4

### NUMBER OF MICE PROTECTED AT TESTED DOSE LEVELS OF FOUR DIFFERENT RATIOS
### OF DOSES OF IBUPROFEN TO DIPHENHYDRAMINE

| Combination Dose Ratio | Dose of Ibuprofen mg/kg | Dose of Diphenhydramine mg/kg | Ibuprofen Equivalent Dose Under Assumption of Additivity mg/kg | Number of Mice Protected | Number of Mice Not Protected |
|---|---|---|---|---|---|
| 19:8 | 8 | 3 | 9.9 | 1 | 7 |
| | 12 | 5 | 15.2 | 3 | 5 |
| | 16 | 7 | 20.4 | 2 | 6 |
| | 28 | 12 | 35.6 | 6 | 2 |
| 9:24 | 3 | 8 | 8.0 | 2 | 6 |
| | 6 | 16 | 16.1 | 2 | 6 |
| | 12 | 32 | 32.2 | 6 | 2 |
| | 15 | 40 | 40.2 | 8 | 0 |
| 6:28 | 3 | 14 | 11.8 | 2 | 6 |
| | 4.5 | 21 | 17.7 | 3 | 5 |
| | 9 | 42 | 35.5 | 7 | 1 |
| 4:32 | 3 | 24 | 18.1 | 2 | 6 |
| | 3.5 | 28 | 21.1 | 3 | 5 |
| | 4.5 | 36 | 27.2 | 6 | 2 |
| | 5 | 40 | 30.2 | 6 | 2 |

EP 0 180 597 B1

## TABLE 5

### ED$_{50}$'s OF COMBINATION TREATMENTS
### IN IBUPROFEN EQUIVALENT DOSES

| Tested Combination Dose Ratios of Ibuprofen to Diphenhydramine | | Ibuprofen Equivalent ED$_{50}$ mg/kg |
| --- | --- | --- |
| I | D | I |
| 100 | 0 | 24 |
| 19 | 8 | 23 |
| 9 | 24 | 18* |
| 6 | 28 | 19* |
| 4 | 32 | 23 |
| 0 | 100 | 24 |

\* ED$_{50}$'s substantially less than 24 mg/kg, the dose that would be expected were the effects additive.

In the pharmaceutical compositions and methods of the present invention, the foregoing active ingredients will be combined with the non-steroidal anti-inflammatory drug(s) and will typically be administered in admixture with suitable pharmaceutical diluents, excipients or carriers (collectively referred to herein as "carrier" materials) suitably selected with respect to the intended form of administration, i.e., oral tablets, capsules, elixirs, syrups, etc. and consistent with conventional pharmaceutical practices. For instance, for oral administration in the form of tablets or capsules, the active drug components may be combined with any oral non-toxic pharmaceutically acceptable inert carrier such as lactose, starch, sucrose, cellulose, magnesium stearate, dicalcium phosphate, calcium sulfate, mannitol, ethyl alcohol (liquid forms) and the like. Moreover, when desired or necessary, suitable binders, lubricants, disintegrating agents and coloring agents can also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, etc. Disintegrators include, without limitation, starch, methylcellulose, agar, bentonite, guar gum, etc. Sweetening and flavoring agents and preservatives can also be included where appropriate.

Of course, additionally, the compositions of the present invention may be formulated in sustained release form to provide the rate controlled release of any one or more of the components to optimize the therapeutic effects, i.e., analgesia, antihistaminic, etc. while minimizing undesirable side effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

As representative suitable formulations consistent with the objects, features and advantages of the present invention, the following non-limiting examples are provided.

Example 2

Ibuprofen - 200 mg

Chlorpheniramine maleate - 8 mg
Phenylpropanolamine hydrochloride - 8 mg
Dextromethorphan hydrobromide - 30 mg
Guaifenesin - 100 mg
Triturate active ingredients and q.s. with lactose to selected capsule size

Example 3

In each fluid ounce:

Naproxen (sodium) 250 mg, dextromethorphan HB 30 mg, phenylpropanolamine hydrochloride 25 mg, orange flavoring and alcohol 10% v/v.

From the foregoing, other typical acceptable pharmaceutical formulations will be apparent to those skilled in the art of pharmaceutical formulations.

While the invention has been described and illustrated with reference to certain preferred embodiments thereof, those skilled in the art will appreciate that various changes, modifications and substitutions can be made therein without departing from the spirit of the invention. For example, effective dosages other than the preferred ranges set forth hereinabove with respect to the active ingredients may be applicable as a consequence of variations of the responsiveness of the mammal treated, severity of symptoms, dosage related adverse effects, if any, observed and similar considerations. Accordingly, such expected variations or differences in the practice of the present invention and the results obtained are contemplated in accordance with the objects and practices of the present invention. It is intended, therefore that the invention be limited only by the scope of the claims which follow.

**Claims**

**Claims for the following Contracting States : BE, CH, LI, DE, FR, GB, IT, LU, NL, SE**

1. A pharmaceutical composition of matter for use in the treatment of cough, cold-like and/or flu symptoms in a mammalian organism, and adapted for unit dosage oral administration, said composition comprising (i) at least one non-narcotic analgesic constituent which is a non-steroidal anti-inflammatory drug (NSAID) selected from a propionic acid derivative, an acetic acid derivative, a fenamic acid derivative, a biphenylcarboxylic acid derivative, an oxicam, or a pharmaceutically acceptable salt thereof (excluding acetylsalicylic acid, acetaminophen and phenacetin), in combinatory admixture with (ii) at least one of (a) at least one antihistamine other than $\alpha$-fluoromethylhistidine, mepyramine or diphenhydramine, (b) at least one sympathomimetic decongestant or bronchodilator, (c) at least one expectorant, and (d) at least one non-narcotic antitussive (cough suppressant), or pharmaceutically acceptable salts thereof.

2. A composition according to Claim 1, wherein the non-steriodal anti-inflammatory drug comprises a propionic acid derivative selected from ibuprofen, naproxen, benoxaprofen, flurbiprofen, fenoprofen, fenbufen, ketoprofen, indoprofen, pirprofen, carprofen, oxaprozin, pranoprofen, miroprofen, trioxaprofen, suprofen, alminoprofen, tiaprofenic acid, fluprofen, or bucloxic acid.

3. A composition according to Claim 2, wherein the amount of said drug comprises between 50 mg to 600 mg in each unit dose thereof.

4. A composition according to Claim 1, wherein the non-steroidal anti-inflammatory drug comprises an acetic acid derivative selected from indomethacin, sulindac, tolmetin, zomepirac, diclofenac, tiopinac, zidometacin, acemetacin, fentiazac, clidanac or oxpinac.

5. A composition according to Claim 4, wherein the amount of said drug ranges between about 25 to 400 mg in each unit dose thereof.

6. A composition according to Claim 1, wherein the non-steroidal anti-inflammatory drug comprises a fenamic acid derivative selected from mefenamic acid, meclofenamic acid, flufenamic acid, niflumic acid or tolfenamic acid.

7. A composition according to Claim 6, wherein the amount of said drug ranges between about 250 to 500 mg in each unit dose thereof.

8. A composition according to Claim 1, wherein the non-steroidal anti-inflammatory drug comprises a biphenylcarboxylic acid derivative selected from diflunisal or flufenisal.

9. A composition according to Claim 8, wherein the amount of said drug ranges between about 250 to 500 mg in each unit dose thereof.

10. A composition according to Claim 1, wherein the non-steroidal anti-inflammatory drug comprises an oxicam selected from piroxicam, sudoxicam, or isoxicam.

11. A composition according to Claim 10, wherein the amount of said drug ranges between about 10 to 20 mg in each unit dose thereof.

12. A composition of matter according to any one of the preceding claims, wherein the antihistamine is selected from chlorpheniramine, brompheniramine, dexchlorpheniramine, dexbrompheniramine, triprolidine, doxylamine, tripelennamine, cyproheptadine, carbinoxamine, bromodiphenydramine, phenyltoloxamine, phenindamine, pyrilamine or azatadine.

13. A composition according to any one of the preceding claims, wherein the decongestant is selected from pseudoephedrine, phenylpropanolamine, or phenylephrine.

14. A composition according to any one of the preceding claims, wherein said antitussive (cough suppressant) is selected from caramiphen, dextromethorphan or codeine.

15. A composition according to any one of the preceding claims, wherein the expectorant is selected from terpin hydrate, guaifenesin, potassium iodide, potassium citrate or potassium guaiacolsulfonate.

16. A composition according to any one of the preceding claims, further comprising a pharmaceutically acceptable non-toxic carrier.

17. A composition according to Claim 16, adapted for oral administration in tablet, capsule or liquid form.

18. Use of a composition comprising (i) at least one non-steroidal anti-inflammatory drug (NSAID) selected from a propionic acid derivative, an acetic acid derivative, a fenamic acid derivative, a biphenylcarboxylic acid derivative, an oxicam, or a pharmaceutically acceptable salt thereof, in combination with (ii) at least one of (a) an antihistamine, (b) a decongestant or bronchodilator, (c) an expectorant and (d) an antitussive (cough suppressant), or pharmaceutically acceptable salt thereof, for the manufacture of a medicament for the treatment of cough, cold-like and/or flu symptoms in a mammalian organism.

**Claims for the following Contracting State : AT**

1. A pharmaceutical composition of matter for use in the treatment of cough, cold-like and/or flu symptoms in a mammalian organism, and adapted for unit dosage oral administration, said composition comprising (i) at least one non-narcotic analgesic constituent which is a non-steroidal anti-inflammatory drug (NSAID) selected from a propionic acid derivative, an acetic acid derivative, a fenamic acid derivative, a biphenylcarboxylic acid derivative, an oxicam, or a pharmaceutically acceptable salt thereof (excluding acetylsalicylic acid, acetaminophen and phenacetin), in combinatory admixture with (ii) at least one of (a) at least one antihistamine other than $\alpha$-fluoromethylhistidine, mepyramine or diphenhydramine, (b) at least one sympathomimetic decongestant or bronchodilator, (c) at least one expectorant, and (d) at least one non-narcotic antitussive (cough suppressant), or pharmaceutically acceptable salts thereof.

2. A composition according to Claim 1, wherein the non-steriodal anti-inflammatory drug comprises a propionic acid derivative selected from ibuprofen, naproxen, benoxaprofen, flurbiprofen, fenoprofen, fenbufen, ketoprofen, indoprofen, pirprofen, carprofen, oxaprozin, pranoprofen, miroprofen, trioxaprofen, suprofen, alminoprofen, tiaprofenic acid, fluprofen, or bucloxic acid.

3. A composition according to Claim 2, wherein the amount of said drug comprises between about 50 mg to 600 mg in each unit dose thereof.

4. A composition according to Claim 1, wherein the non-steroidal anti-inflammatory drug comprises an acetic acid derivative selected from indomethacin, sulindac, tolmetin, zomepirac, diclofenac, tiopinac, zidometacin, acemetacin, fentiazac, clidanac or oxpinac.

5. A composition according to Claim 4, wherein the amount of said drug ranges between about 25 to 400 mg in each unit dose thereof.

6. A composition according to Claim 1, wherein the non-steroidal anti-inflammatory drug comprises a fenamic acid derivative selected from mefenamic acid, meclofenamic acid, flufenamic acid, niflumic acid or tolfenamic acid.

7. A composition according to Claim 6, wherein the amount of said drug ranges between about 250 to 500 mg in each unit dose thereof.

8. A composition according to Claim 1, wherein the non-steroidal anti-inflammatory drug comprises a biphenylcarboxylic acid derivative selected from diflunisal or flufenisal.

9. A composition according to Claim 8, wherein the amount of said drug ranges between about 250 to 500 mg in each unit dose thereof.

10. A composition according to Claim 1, wherein the non-steroidal anti-inflammatory drug comprises an oxicam selected from piroxicam, sudoxicam, or isoxicam.

11. A composition according to Claim 10, wherein the amount of said drug ranges between about 10 to 20 mg in each unit dose thereof.

12. A composition of matter according to any one of the preceding claims, wherein the antihistamine is selected from chlorpheniramine, brompheniramine, dexchlorpheniramine, dexbrompheniramine, triprolidine, doxylamine, tripelennamine, cyproheptadine, carbinoxamine, bromodiphenydramine, phenyltoloxamine, phenindamine, pyrilamine or azatadine.

13. A composition according to any one of the preceding claims, wherein the decongestant is selected from pseudoephedrine, phenylpropanolamine, or phenylephrine.

14. A composition according to any one of the preceding claims, wherein said antitussive (cough suppressant) is selected from caramiphen, dextromethorphan or codeine.

15. A composition according to any one of the preceding claims, wherein the expectorant is selected from terpin hydrate, guaifenesin, potassium iodide, potassium citrate or potassium guaiacolsulfonate.

16. A composition according to any one of the preceding claims, further comprising a pharmaceutically acceptable non-toxic carrier.

17. A composition according to Claim 16, adapted for oral administration in tablet, capsule or liquid form.

18. Use of a composition comprising (i) at least one non-steroidal anti-inflammatory drug (NSAID) selected from a propionic acid derivative, an acetic acid derivative, a fenamic acid derivative, a biphenylcarboxylic acid derivative, an oxicam, or a pharmaceutically acceptable salt thereof, in combination with (ii) at least one of (a) an antihistamine, (b) a decongestant or bronchodilator, (c) an expectorant and (d) an antitussive (cough suppressant), or pharmaceutically acceptable salt thereof, for the manufacture of a medicament for the treatment of cough, cold-like and/or flu symptoms in a mammalian organism.

19. A process for the preparation of a pharmaceutical composition as defined in claim 1, which process comprises combining (i) at least one non-narcotic analgesic constituent which is a non-steroidal anti-inflammatory drug (NSAID) selected from a propionic acid derivative, an acetic acid derivative, a fenamic acid derivative, a biphenylcarboxylic acid derivative, an oxicam, or a pharmaceutically acceptable salt thereof (excluding acetylsalicylic acid, acetaminophen and phenacetin), with (ii) at least one of (a) at least one antihistamine other than α-fluoromethylhistidine, mepyramine or diphenhydramine, (b) at least one sympathomimetic decongestant or bronchodilator, (c) at least one expectorant, and (d) at least one non-narcotic antitussive (cough suppressant), or pharmaceutically acceptable salts thereof, together with a pharmaceutically acceptable carrier or diluent so as to formulate a composition suitable for the treatment of coughs, cold-like and/or flu symptoms.

20. A process according to Claim 19, wherein the non-steriodal anti-inflammatory drug comprises a propionic acid derivative selected from ibuprofen, naproxen, benoxaprofen, flurbiprofen, fenoprofen, fenbufen, ketoprofen, indoprofen, pirprofen, carprofen, oxaprozin, pranoprofen, miroprofen, trioxaprofen, suprofen, alminoprofen, tiaprofenic acid, fluprofen, or bucloxic acid.

21. A process according to Claim 20, wherein the amount of said drug comprises between about 50 mg to 600 mg in each unit dose thereof.

22. A process according to Claim 19, wherein the non-steroidal anti-inflammatory drug comprises an acetic acid derivative selected from indomethacin, sulindac, tolmetin, zomepirac, diclofenac, tiopinac, zidometacin, acemetacin, fentiazac, clidanac or oxpinac.

23. A process according to Claim 22, wherein the amount of said drug ranges between about 25 to 400 mg in each unit dose thereof.

24. A process according to Claim 19, wherein the non-steroidal anti-inflammatory drug comprises a fenamic acid derivative selected from mefenamic acid, meclofenamic acid, flufenamic acid, niflumic acid or tolfenamic acid.

25. A process according to Claim 24, wherein the amount of said drug ranges between about 250 to 500 mg in each unit dose thereof.

26. A process according to Claim 19, wherein the non-steroidal anti-inflammatory drug comprises a biphenylcarboxylic acid derivative selected from diflunisal or flufenisal.

27. A process according to Claim 26, wherein the amount of said drug ranges between about 250 to 500 mg in each unit dose thereof.

28. A process according to Claim 19, wherein the non-steroidal anti-inflammatory drug comprises an oxicam selected from piroxicam, sudoxicam, or isoxicam.

29. A process according to Claim 28, wherein the amount of said drug ranges between about 10 to 20 mg in each unit dose thereof.

30. A process according to any one of claims 19 to 29, wherein the antihistamine is selected from chlorpheniramine, brompheniramine, dexchlorpheniramine, dexbrompheniramine, triprolidine, doxylamine, tripelennamine, cyproheptadine, carbinoxamine, bromodiphenydramine, phenyltoloxamine, phenindamine, pyrilamine or azatadine.

31. A process according to any one of claims 19 to 30, wherein the decongestant is selected from pseudoephedrine, phenylpropanolamine, or phenylephrine.

32. A process according to any one of claims 19 to 31, wherein said antitussive (cough suppressant) is selected from caramiphen, dextromethorphan or codeine.

33. A process according to any one of claims 19 to 32, wherein the expectorant is selected from terpin hydrate, guaifenesin, potassium iodide, potassium citrate or potassium guaiacolsulfonate.

34. A process according to any one of claims 19 to 33, wherein the pharmaceutical composition is formulated for oral administration in tablet, capsule or liquid form.

**Revendications**
**Revendications pour les Etats contractants suivants : BE, CH, LI, DE, FR, GB, IT, LU, NL, SE**

1. Composition de matière pharmaceutique destinée à être utilisée dans le traitement de la toux, des symptômes du type rhume et/ou des symptômes de la grippe dans un organisme de mammifère, et adaptée pour une administration orale sous forme de dosages unitaires, ladite composition comprenant :

   (i) au moins un constituant analgésique non-narcotique qui est un médicament anti-inflammatoire non-stéroïdien (non-steroidal anti-inflammatory drug - NSAID), choisi parmi un dérivé d'acide propionique, un dérivé d'acide acétique, un dérivé d'acide fénamique, un dérivé d'acide biphénylcarboxylique, un oxicam, ou un sel pharmaceutiquement acceptable de ceux-ci (à l'exclusion de l'acide acétylsalicylique, de l'acétaminophène et de la phénacétine), en mélange combiné conjointement avec

   (ii) au moins l'un parmi
      (a) au moins un antihistaminique autre que l'α-fluorométhylhistidine, la mépyramine ou la diphénhydramine,
      (b) au moins un décongestif ou bronchodilalateur sympathomimétique,
      (c) au moins un expectorant, et
      (d) au moins un antitussif non-narcotique (suppresseur de la toux),
   ou leurs sels pharmaceutiquement acceptables.

2. Composition selon la revendication 1, dans laquelle le médicament anti-inflammatoire non-stéroïdien comprend un dérivé d'acide propionique choisi parmi l'ibuprofène, le naproxène, le bénoxaprofène, le flurbiprofène, le fénoprofène, le fenbufène, le cétoprofène, l'indoprofène, le pirprofène, le carprofène, l'oxaprozine, le pranoprofène, le miroprofène, le trioxaprofène, le suprofène, l'alminoprofène, l'acide tiaprofénique, le fluprofène ou l'acide bucloxique.

3. Composition selon la revendication 2, dans laquelle la quantité dudit médicament est comprise entre 50 mg et 600 mg dans chaque dose unitaire de celle-ci.

4. Composition selon la revendication 1, dans laquelle le médicament anti-inflammatoire non-stéroïdien comprend un dérivé d'acide acétique choisi parmi l'indométhacine, le sulindac, la tolmétine, le zomépirac, le diclofénac, le tiopinac, la zidométacine, l'acémétacine, le fentiazac, le clidanac ou l'oxpinac.

5. Composition selon la revendication 4, dans laquelle la quantité dudit médicament se situe entre environ 25 et 400 mg dans chaque dose unitaire de celle-ci.

6. Composition selon la revendication 1, dans laquelle le médicament anti-inflammatoire non-stéroïdien comprend un dérivé d'acide fénamique, choisi parmi l'acide méfénamique, l'acide méclofénamique, l'acide fluflénamique, l'acide niflumique ou l'acide tolfénamique.

7. Composition selon la revendication 6, dans laquelle la quantité dudit médicament se situe entre environ 250 et 500 mg dans chaque dose unitaire de celle-ci.

8. Composition selon la revendication 1, dans lequel le médicament anti-inflammatoire non-stéroïdien comprend un dérivé d'acide biphénylcarboxylique choisi parmi le diflunisal ou le flufénisal.

9. Composition selon la revendication 8, dans laquelle la quantité dudit médicament se situe entre environ 250 et 500 mg dans chaque dose unitaire de celle-ci.

10. Composition selon la revendication 1, dans laquelle le médicament anti-inflammatoire non-stéroïdien comprend un oxicam choisi parmi le piroxicam, le sudoxicam, ou l'isoxicam.

11. Composition selon la revendication 10, dans laquelle la quantité dudit médicament se situe entre environ 10 et 20 mg dans chaque dose unitaire de celle-ci.

12. Composition de matière selon l'une quelconque des revendications précédentes, dans laquelle l'antihistaminique est choisi parmi la chlorphéniramine, la bromphéniramine, la dexchlorphéniramine, la dexbromphéniramine, la triprolidine, la doxylamine, la tripélennamine, la cyproheptadine, la carbinoxamine, la bromodiphénydramine, la phényltoloxamine, la phénindamine, la pyrilamine ou l'azatadine.

18

**13.** Composition selon l'une quelconque des revendications précédentes, dans laquelle le décongestif est choisi parmi la pseudoéphédrine, la phénylpropanolamine ou la phényléphrine.

**14.** Composition selon l'une quelconque des revendications précédentes, dans laquelle ledit antitussif (suppresseur de la toux) est choisi parmi le caramiphène, le dextrométhorphan ou la codéine.

**15.** Composition selon l'une quelconque des revendications précédentes, dans laquelle l'expectorant est choisi parmi l'hydrate de terpine, la guaifénésine, l'iodure de potassium, le citrate de potassium ou le guaicolsulfonate de potassium.

**16.** Composition selon l'une quelconque des revendications précédentes, comprenant en outre un support non-toxique pharmaceutiquement acceptable.

**17.** Composition selon la revendication 16, adaptée pour une administration orale, se présentant sous la forme de comprimés, de capsules ou sous une forme liquide.

**18.** Utilisation d'une composition comprenant :
(i) au moins un médicament anti-inflammatoire non-stéroïdien (NSAID), choisi parmi un dérivé d'acide propionique, un dérivé d'acide acétique, un dérivé d'acide fénamique, un dérivé d'acide biphénylcarboxylique, un oxicam ou un sel pharmaceutiquement acceptable de ceux-ci, en combinaison avec
(ii) au moins l'un parmi
   (a) un antihistaminique,
   (b) un décongestif ou bronchodilatateur,
   (c) un expectorant et
   (d) un antitussif (suppresseur de la toux),
ou leurs sels pharmaceutiquement acceptables,
pour la fabrication d'un médicament pour le traitement de la toux, des symptômes de type rhume et/ou des symptômes de la grippe dans un organisme de mammifère.

**Revendications pour l'Etat contractant suivant : AT**

**1.** Composition de matière pharmaceutique destinée à être utilisée dans le traitement de la toux, des symptômes du type rhume et/ou des symptômes de la grippe dans un organisme de mammifère, et adaptée pour une administration orale sous forme de dosages unitaires, ladite composition comprenant :
(i) au moins un constituant analgésique non-narcotique qui est un médicament anti-inflammatoire non-stéroïdien (non-steroidal anti-inflammatory drug - NSAID), choisi parmi un dérivé d'acide propionique, un dérivé d'acide acétique, un dérivé d'acide fénamique, un dérivé d'acide biphénylcarboxylique, un oxicam, ou un sel pharmaceutiquement acceptable de ceux-ci (à l'exclusion de l'acide acétylsalicylique, de l'acétaminophène et de la phénacétine), en mélange combiné conjointement avec
(ii) au moins l'un parmi
   (a) au moins un antihistaminique autre que l'$\alpha$-fluorométhylhistidine, la mépyramine ou la diphénhydramine,
   (b) au moins un décongestif ou bronchodilalateur sympathomimétique,
   (c) au moins un expectorant, et
   (d) au moins un antitussif non-narcotique (suppresseur de la toux),
ou leurs sels pharmaceutiquement acceptables.

**2.** Composition selon la revendication 1, dans laquelle le médicament anti-inflammatoire non-stéroïdien comprend un dérivé d'acide propionique choisi parmi l'ibuprofène, le naproxène, le bénoxaprofène, le flurbiprofène, le fénoprofène, le fenbufène, le cétoprofène, l'indoprofène, le pirprofène, le carprofène, l'oxaprozine, le pranoprofène, le miroprofène, le trioxaprofène, le suprofène, l'alminoprofène, l'acide tiaprofénique, le fluprofène ou l'acide bucloxique.

**3.** Composition selon la revendication 2, dans laquelle la quantité dudit médicament est comprise entre environ 50 mg et 600 mg dans chaque dose unitaire de celle-ci.

**4.** Composition selon la revendication 1, dans laquelle le médicament anti-inflammatoire non-stéroïdien comprend un dérivé d'acide acétique choisi parmi l'indométhacine, le sulindac, la tolmétine, le zomépirac, le diclofénac, le tiopinac, la zidométacine, l'acémétacine, le fentiazac, le clidanac ou l'oxpinac.

**5.** Composition selon la revendication 4, dans laquelle la quantité dudit médicament se situe entre environ 25 et 400 mg dans chaque dose unitaire de celle-ci.

**6.** Composition selon la revendication 1, dans laquelle le médicament anti-inflammatoire non-stéroïdien comprend un dérivé d'acide fénamique choisi parmi l'acide méfénamique, l'acideméclofénamique, l'acide flufénamique, l'acide niflumique ou l'acide tolfénamique.

**7.** Composition selon la revendication 6, dans laquelle la quantité dudit médicament se situe entre environ 250 et 500 mg dans chaque dose unitaire de celle-ci.

**8.** Composition selon la revendication 1, dans lequel le médicament anti-inflammatoire non-stéroïdien comprend un dérivé d'acide biphénylcarboxylique choisi parmi le diflunisal ou le flufénisal.

**9.** Composition selon la revendication 8, dans laquelle la quantité dudit médicament se situe entre environ 250 et 500 mg dans chaque dose unitaire de celle-ci.

**10.** Composition selon la revendication 1, dans laquelle le médicament anti-inflammatoire non-stéroïdien comprend un oxicam choisi parmi le piroxicam, le sudoxicam ou l'isoxicam.

**11.** Composition selon la revendication 10, dans laquelle la quantité dudit médicament se situe entre environ 10 et 20 mg dans chaque dose unitaire de celle-ci.

**12.** Composition de matière selon l'une quelconque des revendications précédentes, dans laquelle l'antihistaminique est choisi parmi la chlorphéniramine, la brompféniramine, la dexchlorphéniramine, la dex- brompféniramine, la triprolidine, la doxylamine, la tripélennamine, la cyproheptadine, la carbinoxamine, la bromodiphénydramine, la phényltoloxamine, la phénindamine, la pyrilamine ou l'azatadine.

**13.** Composition selon l'une quelconque des revendications précédentes, dans laquelle le décongestif est choisi parmi la pseudoéphédrine, la phénylpropanolamine ou la phényléphrine.

**14.** Composition selon l'une quelconque des revendications précédentes, dans laquelle ledit antitussif (suppresseur de la toux) est choisi parmi le caramiphène, le dextrométhorphan ou la codéine.

**15.** Composition selon l'une quelconque des revendications précédentes, dans laquelle l'expectorant est choisi parmi l'hydrate de terpine, la guaifénésine, l'iodure de potassium, le citrate de potassium ou le guaicolsulfonate de potassium.

**16.** Composition selon l'une quelconque des revendications précédentes, comprenant en outre un support non-toxique pharmaceutiquement acceptable.

**17.** Composition selon la revendication 16, adaptée pour une administration orale se présentant sous la forme de comprimés, de capsules ou sous une forme liquide.

**18.** Utilisation d'une composition comprenant :
   (i) au moins un médicament anti-inflammatoire non-stéroïdien (NSAID), choisi parmi un dérivé d'acide propionique, un dérivé d'acide acétique, un dérivé d'acide fénamique, un dérivé d'acide biphénylcarboxylique, un oxicam ou un sel pharmaceutiquement acceptable de ceux-ci, en combinaison avec
   (ii) au moins l'un parmi
      (a) un antihistaminique,
      (b) un décongestif ou bronchodilatateur,
      (c) un expectorant et
      (d) un antitussif (suppresseur de la toux),

ou leurs sels pharmaceutiquement acceptables,
pour la fabrication d'un médicament pour le traitement de la toux, des symptômes de type rhume et/ou des symptômes de la grippe dans un organisme de mammifère.

19. Procédé de fabrication d'une composition pharmaceutique telle que définie à la revendication 1, lequel procédé comprend la combinaison
(i) d'au moins un constituant analgésique non-narcotique qui est un médicament anti-inflammatoire non-stéroïdien (NSAID), choisi parmi un dérivé d'acide propionique, un dérivé d'acide acétique, un dérivé d'acide fénamique, un dérivé d'acide biphénylcarboxylique, un oxicam, ou un sel pharmaceutiquement acceptable de ceux-ci (à l'exclusion de l'acide acétylsalicylique, de l'acétaminophène et de la phénacétine), avec
(ii) au moins l'un parmi
(a) au moins un antihistaminique autre que l'$\alpha$-fluorométhylhistidine, la mépyramine ou la diphénhydramine,
(b) au moins un décongestif ou bronchodilalateur sympathomimétique,
(c) au moins un expectorant, et
(d) au moins un antitussif non-narcotique (suppresseur de la toux),
ou leurs sels pharmaceutiquement acceptables,
conjointement avec un support ou diluant pharmaceutiquement acceptable, de façon à formuler une composition appropriée pour le traitement des toux, des symptômes de type rhume et/ou des symptômes de la grippe dans un organisme de mammifère.

20. Procédé selon la revendication 19, dans lequel le médicament anti-inflammatoire non-stéroïdien comprend un dérivé d'acide propionique choisi parmi l'ibuprofène, le naproxène, le bénoxaprofène, le flurbiprofène, le fénoprofène, le fenbufène, le cétoprofène, l'indoprofène, le pirprofène, le carprofène, l'oxaprozine, le pranoprofène, le miroprofène, le trioxaprofène, le suprofène, l'alminoprofène, l'acide tiaprofénique, le fluprofène ou l'acide bucloxique.

21. Procédé selon la revendication 20, dans lequel la quantité dudit médicament est comprise entre environ 50 mg et 600 mg dans chaque dose unitaire de celle-ci.

22. Procédé selon la revendication 19, dans lequel le médicament anti-inflammatoire non-stéroïdien comprend un dérivé d'acide acétique choisi parmi l'indométhacine, le sulindac, la tolmétine, le zomépirac, le diclofénac, le tiopinac, la zidométacine, l'acémétacine, le fentiazac, le clidanac ou l'oxpinac.

23. Procédé selon la revendication 22, dans lequel la quantité dudit médicament se situe entre environ 25 et 400 mg dans chaque dose unitaire de celle-ci.

24. Procédé selon la revendication 19, dans lequel le médicament anti-inflammatoire non-stéroïdien comprend un dérivé d'acide fénamique, choisi parmi l'acide méfénamique, l'acide méclofénamique, l'acide fluflénamique, l'acide niflumique ou l'acide tolfénamique.

25. Procédé selon la revendication 24, dans lequel la quantité dudit médicament se situe entre environ 250 et 500 mg dans chaque dose unitaire de celle-ci.

26. Procédé selon la revendication 19, dans lequel le médicament anti-inflammatoire non-stéroïdien comprend un dérivé d'acide biphénylcarboxylique choisi parmi le diflunisal ou le flufénisal.

27. Procédé selon la revendication 26, dans lequel la quantité dudit médicament se situe entre environ 250 et 500 mg dans chaque dose unitaire de celle-ci.

28. Procédé selon la revendication 19, dans lequel le médicament anti-inflammatoire non-stéroïdien comprend un oxicam choisi parmi le piroxicam, le sudoxicam, ou l'isoxicam.

29. Procédé selon la revendication 28, dans lequel la quantité dudit médicament se situe entre environ 10 et 20 mg dans chaque dose unitaire de celle-ci.

**30.** Procédé selon l'une quelconque des revendications 19 à 29, dans lequel l'antihistaminique est choisi parmi la chlorphéniramine, la bromphéniramine, la dexchlorphéniramine, la dexbromphéniramine, la triprolidine, la doxylamine, la tripélennamine, la cyproheptadine, la carbinoxamine, la bromodiphénydramine, la phényltoloxamine, la phénindamine, la pyrilamine ou l'azatadine.

**31.** Procédé selon l'une quelconque des revendications 19 à 30, dans lequel le décongestif est choisi parmi la pseudoéphédrine, la phénylpropanolamine ou la phényléphrine.

**32.** Procédé selon l'une quelconque des revendications 19 à 31, dans lequel ledit antitussif (suppresseur de la toux) est choisi parmi le caramiphène, le dextrométhorphan ou la codéine.

**33.** Procédé selon l'une quelconque des revendications 19 à 32, dans lequel l'expectorant est choisi parmi l'hydrate de terpine, la guaifénésine, l'iodure de potassium, le citrate de potassium ou le guaicolsulfonate de potassium.

**34.** Procédé selon l'une quelconque des revendications 19 à 33, dans lequel la composition pharmaceutique est formulée en vue d'une administration orale se présentant sous la forme de comprimés, de capsules ou sous une forme liquide.

**Patentansprüche**

**Patentansprüche für folgende Vertragsstaaten : BE, CH, LI, DE, FR, GB, IT, LU, NL, SE**

**1.** Pharmazeutische Stoffzusammensetzung zur Verwendung bei der Behandlung von Husten, erkältungsartigen und/oder Grippe-Symptomen im Organismus eines Säugers, und ausgelegt zur oralen Verabreichung in Dosiseinheitsform, welche Zusammensetzung umfaßt: (i) mindestens einen nicht-narkotischen analgetischen Bestandteil, der ein nicht-steroider entzündungshemmender Arzneistoff (non-steroidal anti-inflammatory drug (NSAID)) ist und ausgewählt ist aus einem Propionsäurederivat, einem Essigsäurederivat, einem Fenaminsäurederivat, einem Biphenylcarbonsäurederivat, einem Oxicam oder einem pharmazeutisch akzeptablen Salz davon (exklusive Acetylsalicylsäure, Acetaminophen und Phenacetin), in Kombinationsmischung mit (ii) zumindest einem von (a) mindestens einem Antihistamin, das nicht $\alpha$-Fluormethylhistidin, Mepyramin oder Diphenhydramin ist, (b) mindestens einem sympathomimetischen Dekongestionsmittel oder Bronchodilatator, (c) mindestens einem Expektorans und (d) mindestens einem nicht-narkotischen Antitussivum (Hustendämpfungsmittel), oder pharmazeutisch akzeptable Salze davon.

**2.** Zusammensetzung nach Anspruch 1, worin der nicht-steroide entzündungshemmende Arzneistoff ein Propionsäurederivat ausgewählt aus Ibuprofen, Naproxen, Benoxaprofen, Flurbiprofen, Fenoprofen, Fenbufen, Ketoprofen, Indoprofen, Pirprofen, Carprofen, Oxaprozin, Pranoprofen, Miroprofen, Trioxaprofen, Suprofen, Alminoprofen, Tiaprofensäure, Fluprofen oder Bucloxinsäure umfaßt.

**3.** Zusammensetzung nach Anspruch 2, worin die Menge des Arzneistoffes zwischen 50 mg und 600 mg in jeder Dosiseinheit davon umfaßt.

**4.** Zusammensetzung nach Anspruch 1, worin der nicht-steroide entzündungshemmende Arzneistoff ein Essigsäurederivat ausgewählt aus Indomethacin, Sulindac, Tolmetin, Zomepirac, Diclofenac, Tiopinac, Zidometacin, Acemetacin, Fentiazac, Clidanac oder Oxpinac umfaßt.

**5.** Zusammensetzung nach Anspruch 4, worin die Menge des Arzneistoffes im Bereich zwischen etwa 25 und 400 mg in jeder Dosiseinheit davon liegt.

**6.** Zusammensetzung nach Anspruch 1, worin der nicht-steroide entzündungshemmende Arzneistoff ein Fenaminsäurederivat ausgewählt aus Mefenaminsäure, Meclofenaminsäure, Flufenaminsäure, Nifluminsäure oder Tolfenaminsäure umfaßt.

**7.** Zusammensetzung nach Anspruch 6, worin die Menge des Arzneistoffes in Bereich zwischen etwa 250 und 500 mg in jeder Dosiseinheit davon liegt.

**8.** Zusammensetzung nach Anspruch 1, worin der nicht-steroide entzündungshemmende Arzneistoff ein Biphenylcarbonsäurederivat ausgewählt aus Diflunisal oder Flufenisal umfaßt.

9. Zusammensetzung nach Anspruch 8, worin die Menge des Arzneistoffes im Bereich zwischen etwa 250 und 500 mg in jeder Dosiseinheit davon liegt.

10. Zusammensetzung nach Anspruch 1, worin der nicht-steroide entzündungshemmende Arzneistoff ein Oxicam ausgewählt aus Piroxicam, Sudoxicam oder Isoxicam umfaßt.

11. Zusammensetzung nach Anspruch 10, worin die Menge des Arzneistoffes im Bereich zwischen etwa 10 und 20 mg in jeder Dosiseinheit davon liegt.

12. Stoffzusammensetzung nach einem der vorhergehenden Ansprüche, worin das Antihistamin ausgewählt ist aus Chlorpheniramin, Brompheniramin, Dexchlorpheniramin, Dexbrompheniramin, Triprolidin, Doxylamin, Tripelennamin, Cyproheptadin, Carbinoxamin, Bromdiphenydramin, Phenyltoloxamin, Phenindamin, Pyrilamin oder Azatadin.

13. Zusammensetzung nach einem der vorhergehenden Ansprüche, worin das Dekongestionsmittel ausgewählt ist aus Pseudoephedrin, Phenylpropanolamin oder Phenylephrin.

14. Zusammensetzung nach einem der vorhergehenden Ansprüche, worin das Antitussivum (Hustendämpfungsmittel) ausgewählt ist aus Caramiphen, Dextromethorphan oder Codein.

15. Zusammensetzung nach einem der vorhergehenden Ansprüche, worin das Expectorans ausgewählt ist aus Terpinhydrat, Guaifenesin, Kaliumjodid, Kaliumcitrat oder Kaliumguaiacolsulfonat.

16. Zusammensetzung nach einem der vorhergehenden Ansprüche, welche weiters einen pharmazeutisch akzeptablen nicht-toxischen Träger umfaßt.

17. Zusammensetzung nach Anspruch 16, welche für orale Verabreichung in Tabletten-, Kapsel- oder flüssiger Form ausgelegt ist.

18. Verwendung einer Zusammensetzung, umfassend (i) mindestens einen nicht-steroiden entzündungshemmenden Arzneistoff (NSAID) ausgewählt aus einem Propionsäurederivat, einem Essigsäurederivat, einem Fenaminsäurederivat, einem Biphenylcarbonsäurederivat, einem Oxicam oder einem pharmazeutisch akzeptablen Salz davon, in Kombination mit (ii) mindestens einem von (a) einem Antihistamin, (b) einem Dekongestionsmittel oder Bronchodilatator, (c) einem Expectorans und (d) einem Antitussivum (Hustendämpfungsmittel) oder einem pharmazeutisch akzeptablen Salz davon, zur Herstellung eines Medikaments zur Behandlung von Husten, erkältungsartigen und/oder Grippe-Symptomen im Organismus eines Säugers.

**Patentansprüche für folgenden Vertragsstaat : AT**

1. Pharmazeutische Stoffzusammensetzung zur Verwendung bei der Behandlung von Husten, erkältungsartigen und/oder Grippe-Symptomen im Organismus eines Säugers, und ausgelegt zur oralen Verabreichung in Dosiseinheitsform, welche Zusammensetzung umfaßt: (i) mindestens einen nicht-narkotischen analgetischen Bestandteil, der ein nicht-steroider entzündungshemmender Arzneistoff (non-steroidal anti-inflammatory drug (NSAID)) ist und ausgewählt ist aus einem Propionsäurederivat, einem Essigsäurederivat, einem Fenaminsäurederivat, einem Biphenylcarbonsäurederivat, einem Oxicam oder einem pharmazeutisch akzeptablen Salz davon (exklusive Acetylsalicylsäure, Acetaminophen und Phenacetin), in Kombinationsmischung mit (ii) zumindest einem von (a) mindestens einem Antihistamin, das nicht $\alpha$-Fluormethylhistidin, Mepyramin oder Diphenhydramin ist, (b) mindestens einem sympathomimetischen Dekongestionsmittel oder Bronchodilatator, (c) mindestens einem Expektorans und (d) mindestens einem nicht-narkotischen Antitussivum (Hustendämpfungsmittel), oder pharmazeutisch akzeptable Salze davon.

2. Zusammensetzung nach Anspruch 1, worin der nicht-steroide entzündungshemmende Arzneistoff ein Propionsäurederivat ausgewählt aus Ibuprofen, Naproxen, Benoxaprofen, Flurbiprofen, Fenoprofen, Fenbufen, Ketoprofen, Indoprofen, Pirprofen, Carprofen, Oxaprozin, Pranoprofen, Miroprofen, Trioxaprofen, Suprofen, Alminoprofen, Tiaprofensäure, Fluprofen oder Bucloxinsäure umfaßt.

3. Zusammensetzung nach Anspruch 2, worin die Menge des Arzneistoffes zwischen etwa 50 mg und 600 mg in jeder Dosiseinheit davon umfaßt.

4. Zusammensetzung nach Anspruch 1, worin der nicht-steroide entzündungshemmende Arzneistoff ein Essigsäurederivat ausgewählt aus Indomethacin, Sulindac, Tolmetin, Zomepirac, Diclofenac, Tiopinac, Zidometacin, Acemetacin, Fentiazac, Clidanac oder Oxpinac umfaßt.

5. Zusammensetzung nach Anspruch 4, worin die Menge des Arzneistoffes im Bereich zwischen etwa 25 und 400 mg in jeder Dosiseinheit davon liegt.

6. Zusammensetzung nach Anspruch 1, worin der nicht-steroide entzündungshemmende Arzneistoff ein Fenaminsäurederivat ausgewählt aus Mefenaminsäure, Meclofenaminsäure, Flufenaminsäure, Nifluminsäure oder Tolfenaminsäure umfaßt.

7. Zusammensetzung nach Anspruch 6, worin die Menge des Arzneistoffes im Bereich zwischen etwa 250 und 500 mg in jeder Dosiseinheit davon liegt.

8. Zusammensetzung nach Anspruch 1, worin der nicht-steroide entzündungshemmende Arzneistoff ein Biphenylcarbonsäurederivat ausgewählt aus Diflunisal oder Flufenisal umfaßt.

9. Zusammensetzung nach Anspruch 8, worin die Menge des Arzneistoffes im Bereich zwischen etwa 250 und 500 mg in jeder Dosiseinheit davon liegt.

10. Zusammensetzung nach Anspruch 1, worin der nicht-steroide entzündungshemmende Arzneistoff ein Oxicam ausgewählt aus Piroxicam, Sudoxicam oder Isoxicam umfaßt.

11. Zusammensetzung nach Anspruch 10, worin die Menge des Arzneistoffes im Bereich zwischen etwa 10 und 20 mg in jeder Dosiseinheit davon liegt.

12. Stoffzusammensetzung nach einem der vorhergehenden Ansprüche, worin das Antihistamin ausgewählt ist aus Chlorpheniramin, Brompheniramin, Dexchlorpheniramin, Dexbrompheniramin, Triprolidin, Doxylamin, Tripelennamin, Cyproheptadin, Carbinoxamin, Bromdiphenydramin, Phenyltoloxamin, Phenindamin, Pyrilamin oder Azatadin.

13. Zusammensetzung nach einem der vorhergehenden Ansprüche, worin das Dekongestionsmittel ausgewählt ist aus Pseudoephedrin, Phenylpropanolamin oder Phenylephrin.

14. Zusammensetzung nach einem der vorhergehenden Ansprüche, worin das Antitussivum (Hustendämpfungsmittel) ausgewählt ist aus Caramiphen, Dextromethorphan oder Codein.

15. Zusammensetzung nach einem der vorhergehenden Ansprüche, worin das Expectorans ausgewählt ist aus Terpinhydrat, Guaifenesin, Kaliumjodid, Kaliumcitrat oder Kaliumguaiacolsulfonat.

16. Zusammensetzung nach einem der vorhergehenden Ansprüche, welche weiters einen pharmazeutisch akzeptablen nicht-toxischen Träger umfaßt.

17. Zusammensetzung nach Anspruch 16, welche für orale Verabreichung in Tabletten-, Kapsel- oder flüssiger Form ausgelegt ist.

18. Verwendung einer Zusammensetzung, umfassend (i) mindestens einen nicht-steroiden entzündungshemmenden Arzneistoff (NSAID) ausgewählt aus einem Propionsäurederivat, einem Essigsäurederivat, einem Fenaminsäurederivat, einem Biphenylcarbonsäurederivat, einem Oxicam oder einem pharmazeutisch akzeptablen Salz davon, in Kombination mit (ii) mindestens einem von (a) einem Antihistamin, (b) einem Dekongestionsmittel oder Bronchodilatator, (c) einem Expectorans und (d) einem Antitussivum (Hustendämpfungsmittel) oder einem pharmazeutisch akzeptablen Salz davon, zur Herstellung eines Medikaments zur Behandlung von Husten, erkältungsartigen und/oder Grippe-Symptomen im Organismus eines Säugers.

**19.** Verfahren zur Herstellung einer pharmazeutischen Zusammensetzung wie in Anspruch 1 definiert, welches Verfahren das Vereinigen von (i) mindestens einem nicht-narkotischen analgetischen Bestandteil, der ein nicht-steroider entzündungshemmender Arzneistoff (NSAID) ist und ausgewählt ist aus einem Propionsäurederivat, einem Essigsäurederivat, einem Fenaminsäurederivat, einem Biphenylcarbonsäurederivat, einem Oxicam oder einem pharmazeutisch akzeptablen Salz davon (exklusive Acetylsalicylsäure, Acetaminophen und Phenacetin), mit (ii) zumindest einem von (a) mindestens einem Antihistamin, das nicht α-Fluormethylhistidin, Mepyramin oder Diphenhydramin ist, (b) mindestens einem sympathomimetischen Dekongestionsmittel oder Brochodilatator, (c) mindestens einem Expectorans und (d) mindestens einem nicht-narkotischen Antitussivum (Hustendämpfungsmittel), oder pharmazeutisch akzeptablen Salzen davon, zusammen mit einem pharmazeutisch akzeptablen Träger oder Verdünnungsmittel zur Formulierung einer für die Behandlung von Husten, erkältungsartigen und/oder Grippe-Symptomen geeigneten Zusammensetzung umfaßt.

**20.** Verfahren nach Anspruch 19, worin der nicht-steroide entzündungshemmende Arzneistoff ein Propionsäurederivat ausgewählt aus Ibuprofen, Naproxen, Benoxaprofen, Flurbiprofen, Fenoprofen, Fenbufen, Ketoprofen, Indoprofen, Pirprofen, Carprofen, Oxaprozin, Pranoprofen, Miroprofen, Trioxaprofen, Suprofen, Alminoprofen, Tiaprofensäure, Fluprofen oder Bucloxinsäure umfaßt.

**21.** Verfahren nach Anspruch 20, worin die Menge des Arzneistoffes zwischen etwa 50 mg und 600 mg in jeder Dosiseinheit davon umfaßt.

**22.** Verfahren nach Anspruch 19, worin der nicht-steroide entzündungshemmende Arzneistoff ein Essigsäurederivat ausgewählt aus Indomethacin, Sulindac, Tolmetin, Zomepirac, Diclofenac, Tiopinac, Zidometacin, Acemetacin, Fentiazac, Clidanac oder Oxpinac umfaßt.

**23.** Verfahren nach Anspruch 22, worin die Menge des Arzneistoffes im Bereich zwischen etwa 25 und 400 mg in jeder Dosiseinheit davon liegt.

**24.** Verfahren nach Anspruch 19, worin der nicht-steroide entzündungshemmende Arzneistoff ein Fenaminsäurederivat ausgewählt aus Mefenaminsäure, Meclofenaminsäure, Flufenaminsäure, Nifluminsäure oder Tolfenaminsäure umfaßt.

**25.** Verfahren nach Anspruch 24, worin die Menge des Arzneistoffes im Bereich zwischen etwa 250 und 500 mg in jeder Dosiseinheit davon liegt.

**26.** Verfahren nach Anspruch 19, worin der nicht-steroide entzündrungshemmende Arzneistoff ein Biphenylcarbonsäurederivat ausgewählt aus Diflunisal oder Flufenisal umfaßt.

**27.** Verfahren nach Anspruch 26, worin die Menge des Arzneistoffes im Bereich zwischen etwa 250 und 500 mg in jeder Dosiseinheit davon liegt.

**28.** Verfahren nach Anspruch 19, worin der nicht-steroide entzündungshemmende Arzneistoff ein Oxicam ausgewählt aus Piroxicam, Sudoxicam oder Isoxicam umfaßt.

**29.** Verfahren nach Anspruch 28, worin die Menge des Arzneistoffes im Bereich zwischen etwa 10 und 20 mg pro Dosiseinheit davon liegt.

**30.** Verfahren nach einem der Ansprüche 19 bis 29, worin das Antihistamin ausgewählt ist aus Chlorpheniramin, Brompheniramin, Dexchlorpheniramin, Dexbrompheniramin, Triprolidin, Doxylamin, Tripelennamin, Cyproheptadin, Carbinoxamin, Bromdiphenydramin, Phenyltoloxamin, Phenindamin, Pyrilamin oder Azatadin.

**31.** Verfahren nach einem der Ansprüche 19 bis 30, worin das Dekongestionsmittel ausgewählt ist aus Pseudoephedrin, Phenylpropanolamin oder Phenylephrin.

**32.** Verfahren nach einem der Ansprüche 19 bis 31, worin das Antitussivum (Hustendämpfungsmittel) ausgewählt ist aus Caramiphen, Dextromethorphan oder Codein.

33. Verfahren nach einem der Ansprüche 19 bis 32, worin das Expectorans ausgewählt ist aus Terpinhydrat, Guaifenesin, Kaliumjodid, Kaliumcitrat oder Kaliumguaiacolsulfonat.

34. Verfahren nach einem der Ansprüche 19 bis 33 worin die pharmazeutische Zusammensetzung zur oralen Verabreichung in Tabletten-, Kapsel- oder flüssiger Form formuliert ist.

EP 0 180 597 B1